# EUROPEAN PATENT SPECIFICATION

(11) **EP 1 365 764 B1**
(45) Date of publication and mention of the grant of the patent: **31.08.2005**
(21) Application number: 02703777.9
(22) Date of filing: 27.02.2002
(51) Int. Cl.: A61K 31/545, A61K 9/20, A61K 9/00

(54) **ORAL PHARMACEUTICAL COMPOSITION OF CEFPODOXIME PROXETIL**
ORALE PHARMAZEUTISCHE ZUSAMMENSETZUNG AUS CEFPODOXIM-PROXETIL
COMPOSITION PHARMACEUTIQUE ORALE DE CEFPODOXIME-PROXETIL

(30) Priority: 27.02.2001 IN DE00001900
(43) Date of publication of application: 03.12.2003
(73) Proprietor: RANBAXY LABORATORIES, LTD., New Delhi 110 019 (IN)
(72) Inventor: MALHOTRA, Mukta, New Dehli 110015 (IN); MATHUR, Rajeev Shankar, Delhi 110 052 (IN); MALIK, Rajiv, New Delhi 110 019 (IN)
(74) Representative: Gilholm, Stephen Philip
(86) International application number: PCT/IB2002/000602
(87) International publication number: WO 2002/067943

(56) References cited:
- WO-A-01/37816
- WO-A-01/97851
- US-A- 4 486 425
- US-A- 5 929 086

## Description

### Field of the Invention

The present invention relates to a stable pharmaceutical composition of cefpodoxime proxetil, for oral administration.

### Background of the Invention

Cefpodoxime proxetil, chemically known as [6R-[6α, 7β(Z)]]-7-[[(2-amino-4-thiazolyl)(methoxyimino)acetyl]amino]-3-(methoxymethyl)-8-oxo-5-thia-1-azabicyclo[4.2.0] oct-2-ene-2-carboxylic acid 1- [[(1-methylethoxy)carbonyl]oxy]ethyl ester, and its pharmaceutically acceptable acid addition salts has been described in U.S. Patent No. 4,486,425.

Cefpodoxime proxetil is an orally administered prodrug which is absorbed and de-esterified by the intestinal mucosa to release its active metabolite, the third generation cephalosporin, cefpodoxime. Cefpodoxime is stable towards hydrolysis by the most commonly found plasmid-mediated β-lactamases. The drug has a broad spectrum of antibacterial activity, encompassing both gram-negative and gram-positive bacteria while sharing the familiar and relatively benign tolerability profile of other broad spectrum cephalosporins with regard to both the incidence and severity of adverse events. Thus, rendering it as an effective alternative to currently used β-lactams for empirical therapy in a wide range of community acquired infections in both adult and paediatric patients.

However, the therapeutic efficacy of any drug depends to a considerable extent on the design of its pharmaceutical formulation. It is important that cephalosporin compounds for oral administration should be in a form which provides high bioavailability whereby absorption of the antibiotic into the blood stream is maximized and the amount of the antibiotic remaining in the gastro-intestinal tract is minimized.

The physico-chemical attributes and bio-pharmacological characteristics accounts for the formulation of a bioavailable pharmaceutical composition. The wettability and dissolution properties of a biologically active substance or drug precursor greatly influence the bioavailability.

Cefpodoxime proxetil is hydrophobic in nature having contact angle with water greater than 95º. Further, it has been found that cefpodoxime exhibits gel formation and forms a gelatinous mass when in contact with aqueous media (Hamaura T. et.al., S.T.P. Pharma Sciences, 1995; 5(4):324). The gel formation results in poor disintegration and slow dissolution and hence the absorption of cefpodoxime from the gastro-intestinal tract is greatly reduced.

The literature discloses various approaches to obviate problems related to unfavourable absorption characteristics.

One such approach mentioned in the prior art include micronization of an active principle resulting in improved dissolution characteristics and better bioavailability. High speed running mills or air-jet mills are used to reduce the particle size to a range of 5-10 microns of low solubility drugs. However, sizing is not the panacea for all such problems as it is well ascertainable by those skilled in the formulation development that such grinding methods results in the tendency of the milled particles to develop electrostatic charges which leads to agglomeration, impeded flow and poor wetting. These disadvantages at times negate the very objective of comminution. (Aguiar et. al., J.Pharm. Sci., 1969; 56(10):1243).

Other techniques which have been described in the prior art for enhancing the aqueous solubility of the drug include formation of "inclusion compounds" by their complexation with solubility enhancers such as cyclodextrins. U.S. Patent No. 5,646,131 relates to a method for enhancing the solubilization of an insoluble or sparingly soluble drug using cyclodextrin together with a hydroxycarboxylic or polycarboxylic acid. While such an approach may be suitable for the low solubility β-lactam antibiotic drugs such as amoxicillin, however, it would be highly unsuitable for rapidly gelling drugs such as cefpodoxime proxetil.

Still other techniques are directed towards rapidly disintegrating cores to alleviate the gel formation. U.S. Pat. No. 4,897,270 discloses that cefuroxime axetil when in contact with aqueous media forms a gelatinous mass even at physiological temperatures. The inventors have addressed this problem by formulating a film coated pharmaceutical tablet wherein the film coat ruptures very rapidly (less than 40 seconds in 0.07M hydrochloric acid at 37ºC) and the core then immediately disintegrates, allowing dispersion and dissolution of cefuroxime axetil before any gelling effect can occur. It is well recognized by those skilled in the art that it may be difficult to extend this technique to all drugs having the tendency to form a gelatinous mass upon contact with aqueous media.

As aforementioned, several pharmaceutical compositions are described in the reference literature which relate to means to improve absorption and thus bioavailability profile of drugs. However, as heretofore disclosed, cefpodoxime proxetil is a highly hydrophobic drug that has a tendency to form a gel in aqueous media which thereby results in slow dissolution and hence poor bioavailability. It is therefore necessary that the pharmaceutical composition is formulated such that bridging of molecules to form a gel is prevented and thereby, the dissolution is improved.

### Summary of the Invention

It is an objective of the present invention to provide a pharmaceutical composition for oral administration wherein the formation of gelatinuous mass is hindered and wettability of cefpodoxime is enhanced thereby ensuring an improved rate of dissolution and better bioavailability.

An additional objective of the present invention is to provide cefpodoxime proxetil adsorbates which are incorporated in pharmaceutical compositions for preparing various dosage forms and also the use thereof.

In keeping with these objectives, the present invention provides a pharmaceutical composition of cefpodoxime proxetil, which effects readier dissolution of the drug and better bioavailability. As embodied and described herein, the present invention resides in a pharmaceutical composition for oral administration which comprises pharmaceutically effective amount of cefpodoxime proxetil adsorbed on to a pharmaceutical carrier wherein the drug have a mean particle size of less than 30 microns and a particle size distribution such that at least 90% of the particles are less than 75 microns and the surface area is greater than 1.3 m²/g.

The present invention also comprehends a pharmaceutical composition in the form of beads, pellets, granules, tablets, capsules and suspension incorporating cefpodoxime proxetil in the carrier and optional pharmaceutical adjuvants such as inert diluents, fillers, and the like. Also, the pharmaceutical composition in solid dosage form may be optionally coated with a rapidly dissolving water soluble polymer film coat.

### Detailed Description of the Invention

The present invention is directed to a pharmaceutical composition exhibiting enhanced dissolution and bioavailability of cefpodoxime proxetil which is attained through adsorption of the micronized drug on to a pharmaceutical carrier to form cefpodoxime - carrier adsorbates. By "adsorbed" it is meant that the drug particles are closely associated with the carrier in clusters but not all the drug particles may actually be in contact with the carrier particle. This is distinguished from simple mixtures, where the drug particles and the carrier particles often lie independently in a non-homogenous or homogenous manner.

The fine particle size and surface area of the adsorbed cefpodoxime is critical to the present invention wherein the particle size of the adsorbed drug is such that the mean particle size is less than 30 microns and the distribution of particle size is such that at least 90% of the particles are less than 75 microns and the surface area is greater than 1.3 m²/g. Preferably, the particle size of the adsorbed drug is such that the mean particle size is less than 20 microns and a particle size distribution such that at least 90% of the particles are less than 50 microns and the surface area is greater than 3.0 m²/g.

The adsorption of the drug on the carrier results in an increase in the wettability of cefpodoxime as a consequence of the very large dispersion of it on the network of hydrophilic pharmaceutical carrier which also acts as a channeling agent. Also, these agents swell remarkably upon ingress of water and thereby disintegrate the gel layers that may be formed around the drug particles resulting in improved dissolution and hence better bioavailability.

The composition according to the present invention comprises a pharmaceutical carrier which constitutes hydrophilic polymer in conjunction with other pharmaceutically acceptable adjuvants which together regulate the dissolution and release of cefpodoxime. The polymers which are amenable to the antibacterial therapy utilizing the novel pharmaceutical composition of the present invention include any of those suitable for oral administration. The hydrophilic polymer forming the pharmaceutical carrier in accordance with this invention is any such polymer that is non-toxic, swells upon imbibition of water and provides for improved dissolution of cefpodoxime. The hydrophilicity of these polymers facilitate the drug-carrier adsorbate to swell upon ingress of water and thereby hinder the formation of gelatinous mass. These hydrophilic polymers may be used individually or in combination. Examples of polymers suitable for this invention include the polymers well known in the pharmaceutical art and may be selected from the group comprising cellulose ethers such as hydroxypropyl methylcelluloses of different grades, hydroxypropyl cellulose of different grades, hydroxyethyl cellulose, methyl cellulose, hydroxypropyl ethylcellulose, hydroxyethyl methylcellulose, carboxymethyl cellulose, calcium carboxymethyl cellulose, sodium carboxymethyl cellulose, hydroxycellulose and the like; acrylic polymers such as Eudragit RS 30D, Eudragit RL 30D, Eudragit NE 30 D, Eudragit RSPO, polyacrylic polymers such as available under the brand name carbopol; natural gums as xanthan gum, karaya gum, locust bean gum, sodium alginate, alginic acid, guar gum, gelan gum, gum arabic, tragacanth, carrageenan, pectin, agar and the like.

The amount of polymer relative to the drug may vary depending on the nature of polymers, their physiochemical characteristics and other adjuvants that may be present as the integral part of the formulation. However, the polymers may be present in an amount from about 1% to about 60% weight, more preferably from about 1% to 50% by weight of the total weight of the pharmaceutical composition.

In accordance with this invention, the pharmaceutical composition may contain a surface active agent to facilitate the wettability and dissolution of the drug. The surfactant used may be selected from those conventionally used in pharmaceutical preparations such as sodium lauryl sulphate, polyoxyethylene - polyoxypropylene copolymers (Poloxamer), polysorbates (such as available as Tween 20, Tween 40, Tween 60 and the like), sorbitan esters (such as sorbitan monolaurate, sorbitan monopalmitate and the like), and the like. The composition of the invention may contain surface active agent in an amount from about 1% to about 5%, and preferably from about 1.5% to about 3.5% by weight of the total weight of the composition.

According to the present invention, the compositions may contain a swelling agent from the class of compounds commonly known as superdisintegrants which absorb large amounts of fluid to swell significantly and the swelling pressure so generated causes the hydrated gelatinous layers to consequently disintegrate even prior to its formation. Examples of swelling agents that may be used in the present invention include cross-linked polyvinylpyrrolidone, cross-linked carboxymethyl cellulose sodium, sodium starch glycolate, and the like. The swelling agent may be present in an amount from about 3% to about 6%, by weight of the total weight of the composition.

Optionally, there may also be incorporated into the pharmaceutical composition of the present invention other conventional pharmaceutically acceptable adjuvants known in the art of formulation development. The present invention is not to be construed as being limited to any particular excipient or class of pharmaceutical excipients. The adjuvants selected should be such that there is no interaction which would substantially reduce the pharmaceutical efficacy of the composition of the present invention. Pharmaceutical adjuvants used must be of high purity and low toxicity to render them suitable for administration. The choice of these adjuvants and the amounts to be used are considered to be within the purview of one skilled in the art and would depend on the type of dosage form.

The pharmaceutical composition may contain one or more of a water soluble and /or water dispersible diluent. Examples of water soluble diluents that may be used in the present invention include, but are not limited to lactose, calcium sulphate, mannitol, dextrates, dextrin, dextrose, sucrose and the like. Water dispersible diluents which refer to water insoluble pharmaceutical excipients that disperse readily in water include but are not limited to, cellulose based excipients such as microcrystalline cellulose, powdered cellulose, starches such as corn starch, pregelatinized starch, clays or clay minerals such as kaolin, bentonite, attapulgite, and the like.

According to the present invention the pharmaceutical composition may be prepared either in the form of pellets, beads, granules, tablets, dry syrup, suspension and capsules.

In those embodiments of the present invention wherein the pharmaceutical composition is in the form of solid dosage form, it may contain in addition to the above ingredients, pharmaceutical grade magnesium stearate or stearic acid, and the like as glidant, talc and the like as an anti-adherent and silicon dioxide or hydrogenated vegetable oil, and the like as a lubricant.

The pharmaceutical composition in accordance to the present invention may be optionally coated with a rapidly dissolving water soluble film coat. The examples of water soluble polymers include hydroxypropyl methylcellulose, hyroxypropyl cellulose, and the like. The solid unit dosage form in accordance with the present invention may be coated to a weight build-up of about 1 % to about 10% by weight, preferably from about 1% to about 4% by weight, of the total weight of the composition.

In embodiments of the present invention wherein the pharmaceutical composition is in the form of suspension or dry syrup, it may also contain other adjuvants recognized in the art of pharmaceutical compounding such as citric acid and sodium citrate, and the like as buffering agents, sodium benzoate and the like as preservative, colloidal silicon dioxide, and the like as anticaking agent, flavours to mask bitter taste, xanthan gum, carageenan, and the like as suspending agents, simethicone, and the like as antifoaming agents and ferric oxide, and the like as colouring agents.

In those embodiments of the present invention wherein the pharmaceutical composition is in the form of capsule dosage form, the capsule shell may be of a hard gelatin or a soft gelatin type. Furthermore, the capsules made of starch or hydroxypropyl methylcellulose may also be used.

Cefpodoxime proxetil is subjected to micronization using milling apparatus whereby it is reduced to a very fine powder due to attrition of the particles by collisions between particles and between particles and machine surfaces. Its micronization may be advantageously carried out in an accelerated air-jet mill wherein collision of drug particles with each other under a high pressure stream of air causes reduction of particle size and increases the specific surface area of the material, manifold. The period of milling may vary depending on the size of the mill, the velocity of the air, the type of feed material and the quantity of feed material. The effects of these variables are well known in the art and the present invention may be worked over a range of these variables. However, the comminution operation may be carried out until the powder obtained is such that the mean particle size is less than 30 microns and a particle size distribution such that at least 90% of the particles are less than 75 microns and the surface area is greater than 1.3m²/g.

In accordance with the present invention, the micronized drug is loaded onto the pharmaceutical carrier under the direct, high velocity collision force, that is, adsorption through impaction. For the purpose, any suitable apparatus that utilizes the crushing action under force between two surfaces, may be employed. Accordingly, milling machines that work on substantially the same principle may be used. Examples of such milling machines include various makes of rotating ball mill, vibrational ball mill, automatic mortar mill, roller mill, gyratory mill, comminuting mill and the like. In the preferred embodiment of the invention, a mill such as comminuting mill with a hammer forward configuration may be used. The principle of operation of this mill is one of high-velocity impact between rapidly moving hammers mounted on a rotor and the powdered particles. As the hammer hits the drug and the carrier blend, particles of the drug get loaded on to the carrier due to the impact generated by the same. This loads the drug well on to the carrier that warrant further increase in surface area and better wettability and dissolution profile. The period of loading may vary depending on the size of the mill, the speed of rotation of the main shaft, type of feed material and quantity of feed material. The effects of these variables are well known in the art and the invention may be worked over a range of these variables.

According to the present invention, the pharmaceutical composition is prepared by blending cefpodoxime-carrier adsorbates with pharmaceutically acceptable adjuvants such as fillers, inert diluents, and the like. The blend is directly compressed into tablets or may be filled into capsules or bottles as dry syrup. Alternatively, the pharmaceutical composition is prepared by blending the aforementioned ingredients with cefpodoxime-carrier adsorbates. The blend is roll compacted and then sized to obtain granules. The granules may be filled into capsules or bottles as dry syrup or compressed into tablets. Alternatively, the pharmaceutical composition is prepared by blending cefpodoxime-carrier adsorbates with above mentioned ingredients and granulating the same with a solution of binder. The granules so obtained are dried, sized and may be filled into capsules or bottles as dry syrup or compressed into tablets.

In those embodiments of the present invention wherein the foregoing composition is in the form of spherical pellets or beads, the art of producing such dosage forms by extrusion and spheronisation techniques or techniques based on high shear granulation or fludized bed techniques may be used. Single unit pellets can be produced on industrial scale using lozenge and troches cutting machines.

The following examples further illustrate this invention and are not to be construed as limiting the scope but read in conjunction with the description above, provide further understanding of the present invention and an outline of the process for preparing the composition of the invention.

### EXAMPLE 1

This example illustrates the present invention in the form of a tablets using micronized active ingredient. Dry granulation was used as the processing technique for the preparation of oral formulation. The pharmaceutical composition is given in Table 1.

**Table 1**

| **Ingredients** | **%w/w** |
|---|---|
| Cefpodoxime Proxetil | 49.79 |
| Calcium carboxymethyl cellulose | 42.07 |
| Lactose | 3.64 |
| Hydroxypropyl cellulose, low viscosity | 1.64 |
| Sodium Lauryl Sulphate | 2.05 |
| Magnesium Stearate | 0.82 |

Cefpodoxime proxetil was micronized using fluid energy mill with a compressed air pressure of about 5 PSIG and the particle size distribution was achieved such that about 90% of the particles are less than 50 microns and having a surface area of about 5.55m²/g.

Micronized cefpodoxime proxetil was blended with calcium carboxymethyl cellulose and sodium lauryl sulphate and sifted through a sieve (British Standard Sieve (BSS) 25; 600 µm). The blend was passed through a comminuting mill using 1.0 mm screen and impact forward configuration at high speed. Lactose and hydroxypropyl cellulose (low viscosity) were sieved through 600 µm mesh and mixed with the milled blend in a non-shear blender (octagonal blender). The blend so obtained was dry granulated by compaction. The compacts were sized through 710 µm mesh (British Standard Sieve (BSS) 22) to obtain granules. The sized granules were blended with magnesium stearate (sieved through British Standard Sieve (BSS) 44; 355 µm mesh) prior to compression into tablets.

The tablets were tested for drug release in 900 ml of glycine buffer of pH 3.0 using USP apparatus 2 with paddle speed at 75 rpm. The samples of the media were periodically withdrawn and spectrophotometrically analyzed for cefpodoxime content at 259 nm. The dissolution results are given in Table 2.

**Table 2**

| **Time (Minutes)** | **Cumulative percent cefpodoxime released** |
|---|---|
| 10 | 88.8 |
| 20 | 101.7 |
| 30 | 104.3 |

### EXAMPLE 2

This example illustrates the process for the preparation of dry syrup of cefpodoxime proxetil. The pharmaceutical composition is given in Table 3.

**Table 3**

| **Ingredients** | **Quantity (mg/5ml)** |
|---|---|
| Cefpodoxime Proxetil | 68.46 |
| Microcrystalline cellulose and carboxymethyl cellulose sodium (Avidel RC-591) | 25.00 |
| Hydroxypropyl cellulose, low viscosity | 20.00 |
| Croscarmellose sodium | 143.50 |
| Lactose | 6.54 |
| Citric acid | 6.00 |
| Sodium citrate | 0.70 |
| Sodium benzoate | 10.00 |
| Colloidal silicon dioxide | 16.25 |
| Carrageenan | 6.00 |
| Sucrose | 2669.80 |
| Flavours | Quantity sufficient |
| Colouring agent | Quantity sufficient |

Cefpodoxime proxetil was micronized using fluid energy mill as described in Example 1. Micronized cefpodoxime proxetil was blended with dried croscarmellose sodium, coprocessed microcrystalline cellulose and carboxymethyl cellulose sodium and hydroxypropyl cellulose and sifted through a sieve (British Standard Sieve (BSS) 25; 600 microns). The blend was passed through comminuting mill using 1.0 mm screen and impact forward configuration at high speed. Dried sucrose and colouring agent were sieved through 600 µm mesh (British Standard Sieve (BSS) 25) and 150 µm mesh (British Standard Sieve (BSS) 100), respectively. Citric acid, sodium citrate, colloidal silicon dioxide and sodium benzoate were sieved through 710 µm mesh (British Standard Sieve (BSS) 22) and blended with a portion of dried sucrose and ferric oxide. This mixture was also passed through the comminuting mill. Lactose, flavours and carrageenan were sieved through 710 microns mesh and blended with the aforementioned milled blends and remaining sucrose in geometric serial dilutions in a non-shear blender prior to its filling in bottles.

The suspension blend was characterized for drug release in glycine buffer as described in Example 1 and the dissolution results are recorded in Table 4.

**Table 4**

| **Time (Minutes)** | **Cumulative percent cefpodoxime released** |
|---|---|
| 10 | 102.0 |
| 20 | 104.0 |
| 30 | 104.0 |

### EXAMPLE 3

The example illustrates the process for the preparation of suspension dosage form of cefpodoxime proxetil having a composition as given in Table 5.

**Table 5**

| **Ingredients** | **Quantity (mg/5ml)** |
|---|---|
| Cefpodoxime Proxetil | 68.46 |
| Hydroxypropyl cellulose, low viscosity | 20.00 |
| Croscarmellose sodium | 20.00 |
| Sucrose | 2814.34 |
| Simethicone emulsion | 25.00 |
| Citric acid | 6.00 |
| Sodium citrate | 0.70 |
| Xanthan Gum | 9.00 |
| Colloidal Silicon Dioxide | 16.25 |
| Sodium Benzoate | 10.00 |
| Flavours | Quantity sufficient |
| Colouring agent | Quantity sufficient |

Cefpodoxime proxetil was micronized using fluid energy mill as disclosed in Example 1. Croscarmellose sodium, hydroxypropyl cellulose and a portion of dried sucrose were sieved through 710 µm mesh (British Standard Sieve (BSS) 22) and blended in a high shear mixer. The blend was granulated with simethicone emulsion and dried to obtain granules. Micronized cefpodoxime proxetil, sodium benzoate, citric acid, sodium citrate, colloidal silicon dioxide, xanthan gum, flavours and granular carrier prepared above were sifted through a 600 microns mesh (British Standard Sieve (BSS) 25) and blended with colour (sieved through British Standard Sieve (BSS) 100, 150 microns). This blend was passed through comminuting mill using 1.0 mm screen and impact forward configuration at high speed. The material so obtained was blended with the remaining sucrose in geometric serial dilutions in a non-shear blender prior to its filling in the bottles.

The granular suspension was characterized for drug release as disclosed in Example 1 and the dissolution profile is tabulated in Table 6.

**Table 6**

| **Time (Minutes)** | **Cumulative percent cefpodoxime released** |
|---|---|
| 10 | 99.43 |
| 20 | 104.45 |
| 30 | 104.26 |

### EXAMPLE 4

This example illustrates the present invention in the form of tablets wherein wet granulation is used as the processing technique. The pharmaceutical composition is given in Table 7.

**Table 7**

| **Ingredients** | **%w/w** |
|---|---|
| Cefpodoxime Proxetil | 43.47 |
| Calcium carboxymethyl cellulose | 47.08 |
| Lactose | 3.33 |
| Calcium carbonate | 2.00 |
| Hydroxypropyl cellulose, low viscosity | 1.50 |
| Sodium Lauryl Sulphate | 1.88 |
| Magnesium Stearate | 0.75 |

Cefpodoxime proxetil was sized using fluid energy mill as described in Example 1. Micronized cefpodoxime proxetil was blended with calcium carboxymethyl cellulose, lactose, calcium carbonate and sodium lauryl sulphate and sieved through 710 µm mesh (British Standard Sieve (BSS) 22). The blend so obtained was granulated with the solution of hydroxypropyl cellulose in water. The granules were dried and passed through 710 µm mesh. The sized granules were blended with magnesium stearate, previously sieved through 355 µm mesh (British Standard Sieve (BSS) 44) and compressed into tablets.

The tablets were tested for dissolution profile as disclosed in Example 1 and the results are recorded in Table 8.

**Table 8**

| **Time (Minutes)** | **Cumulative percent cefpodoxime released** |
|---|---|
| 10 | 50.4 |
| 20 | 79.8 |
| 30 | 89.9 |

## Claims

1. A pharmaceutical composition for oral administration which comprises pharmaceutically effective amount of cefpodoxime proxetil adsorbed on to a pharmaceutical carrier wherein the cefpodoxime proxetil has a mean particle size of less than 30 microns and a particle size distribution such that at least 90% of the particles are less than 75 microns and the surface area is greater than 1.3 m²/g.

2. The pharmaceutical composition according to claim 1 wherein the cefpodoxime proxetil has a mean particle size of less than 20 microns and a particle size distribution such that at least 90% of the particles are less than 50 microns and the surface area is greater than 3.0 m²/g.

3. The phramaceutical composition according to claim 1 wherein the pharmaceutical carrier is a hydrophilic polymer.

4. The pharmaceutical composition according to claim 3 wherein the hydrophilic polymer is a cellulose ether, acrylic polymer, natural gum, and mixtures thereof.

5. The pharmaceutical composition according to claim 4 wherein the cellulose ether is selected from the group consisting of hydroxypropyl cellulose, hydroxypropyl methylcellulose, hydroxyethyl cellulose, methylcellulose, hydroxyethyl methylcellulose, hydroxypropyl ethylcellulose, carboxymethyl cellulose, calcium carboxymethyl cellulose, sodium carboxymethyl cellulose hydroxycellulose, and mixtures thereof.

6. The pharmaceutical composition according to claim 4 wherein the acrylic polymer is selected from the group consisting of methacrylates, polyacrylate copolymers, and mixtures thereof.

7. The pharmaceutical composition according to claim 4 wherein the natural gum is selected from the group consisting of xanthan gum, karaya gum, locust bean gum, guar gum, gelan gum, gum arabic, tragacanth, carrageenan, pectin, agar, alginic acid, sodium alginate, and mixtures thereof.

8. The pharmaceutical composition according to claim 1 wherein the polymer comprises about 1% to about 60% by weight of said composition.

9. The pharmaceutical composition according to claim 1 wherein the polymer comprises about 1% to about 50% by weight of said composition.

10. The pharmaceutical composition according to claim 1 wherein the pharmaceutical carrier further comprises a surface active agent.

11. The pharmaceutical composition according to claim 10 wherein the surface active agent is selected from the group consisting of sodium lauryl sulphate, polyoxyethylene-polyoxypropylene copolymers, polysorbates, sorbitan esters, and mixtures thereof.

12. The pharmaceutical composition according to claim 1 wherein the surface active agent comprises about 1% to about 5% by weight of said composition.

13. The pharmaceutical composition according to claim 1 wherein the composition also comprises swelling agents.

14. The pharmaceutical composition according to claim 13 wherein the swelling agent comprises a superdisintegrant.

15. The pharmaceutical composition according to claim 14 wherein the swelling agent is selected from the group consisting of cross-linked polyvinyl pyrrolidone, cross-linked sodium carboxymethyl cellulose, sodium starch glycolate, and mixtures thereof.

16. The pharmaceutical composition according to claim 1 wherein the swelling agent comprises about 3% to about 6% by weight of said composition.

17. The pharmaceutical composition according to claim 1 wherein the composition further comprises a diluent.

18. The pharmaceutical composition according to any one of the preceding claims wherein the composition is being formed into a physical form selected from the group consisting of pellets, beads, granules, tablets, dry syrup, suspension and capsules.

19. The pharmaceutical composition according to claim 18 wherein the solid dosage form further comprises binder, glidant, anti-adherent, lubricant, and mixtures thereof.

20. The pharmaceutical composition according to claim 18 wherein the tablet dosage form further comprises coating with a fast dissolving film of a water soluble polymer.

21. The pharmaceutical composition according to claim 18 wherein the dry syrup or suspension dosage form further comprises buffering agent, preservative, anticaking agent, suspending agent, antifoaming agent, colouring agent flavour, and mixtures thereof.

22. The pharmaceutical composition according to claim 18 wherein the capsule shell is made of gelatin, hydroxypropyl methylcellulose or starch.

## Patentansprüche

1. Pharmazeutische Zusammensetzung für orale Verabreichung, welche eine pharmazeutisch wirksame Menge von Cefpodoxim-Proxetil aufweist, welche auf einem pharmazeutischen Träger adsorbiert ist, wobei das Cefpodoxim-Proxetil eine mittlere Teilchengröße von weniger als 30 Mikrometer und eine derartige Teilchengrößenverteilung aufweist, dass wenigstens 90% der Teilchen kleiner als 75 Mikrometer sind und die Oberfläche größer als 1,3 m²/g ist.

2. Pharmazeutische Zusammensetzung nach Anspruch 1, wobei das Cefpodoxim-Proxetil eine mittlere Teilchengröße von weniger als 20 Mikrometer und eine derartige Teilchengrößenverteilung aufweist, dass zumindest weniger als 90% der Teilchen kleiner als 50 Mikrometer sind und die Oberfläche größer als 3,0 m²/g ist.

3. Pharmazeutische Zusammensetzung nach Anspruch 1, wobei der pharmazeutische Träger ein hydrophiles Polymer ist.

4. Pharmazeutische Zusammensetzung nach Anspruch 3, wobei das hydrophile Polymer ein Celluloseether, Acrylpolymer, natürlicher Gummi und eine Mischung derselben ist.

5. Pharmazeutische Zusammensetzung nach Anspruch 4, wobei der Celluloseether ausgewählt ist aus der Gruppe bestehend aus Hydroxypropylcellulose, Hydroxypropylmethylcellulose, Hydroxyethylcellulose, Methylcellulose, Hydroxyethylmethylcellulose, Hydroxypropylethylcellulose, Carboxymethylcellulose, Calciumcarboxymethylcellulose, Natriumcarboxymethylcellulose Hydroxycellulose und Mischungen derselben.

6. Pharmazeutische Zusammensetzung nach Anspruch 4, wobei das Acrylpolymer ausgewählt ist aus der Gruppe bestehend aus Methacrylate, Polyacrylat-Copolymere und Mischungen derselben.

7. Pharmazeutische Zusammensetzung nach Anspruch 4, wobei der natürliche Gummi ausgewählt ist aus der Gruppe bestehend aus Xanthangummi, Karayagummi, Johannisbrotgummi, Guargummi, Gelangummi, Gummi arabicum, Tragacanth, Carrageenan, Pectin, Agar, Algininsäure, Natriumalginat und Mischungen derselben.

8. Pharmazeutische Zusammensetzung nach Anspruch 1, wobei das Polymer ungefähr 1 Gew.-% bis ungefähr 60 Gew.-% der Zusammensetzung ausmacht.

9. Pharmazeutische Zusammensetzung nach Anspruch 1, wobei das Polymer ungefähr 1 Gew.-% bis ungefähr 50 Gew.-% der Zusammensetzung ausmacht.

10. Pharmazeutische Zusammensetzung nach Anspruch 1, wobei der pharmazeutische Träger ferner ein oberflächenaktives Mittel enthält.

11. Pharmazeutische Zusammensetzung nach Anspruch 10, wobei das oberflächenaktive Mittel ausgewählt ist aus der Gruppe bestehend aus Natriumlaurylsulfat, Polyoxyethylen-Polyoxypropylen-Copolymere, Polysorbate, Sorbitanester und Mischungen derselben.

12. Pharmazeutische Zusammensetzung nach Anspruch 1, wobei das oberflächenaktive Mittel ungefähr 1 Gew.-% bis ungefähr 5 Gew.-% der Zusammensetzung ausmacht.

13. Pharmazeutische Zusammensetzung nach Anspruch 1, wobei die Zusammensetzung des Weiteren Quellungsmittel enthält.

14. Pharmazeutische Zusammensetzung nach Anspruch 13, wobei das Quellungsmittel ein Superabbaumittel enthält.

15. Pharmazeutische Zusammensetzung nach Anspruch 14, wobei das Quellungsmittel ausgewählt ist aus der Gruppe bestehend aus vernetztem Polyvinylpyrrolidon, vernetzter Natriumcarboxymethylcellulose, Natriumstärkeglykolat und Mischungen derselben.

16. Pharmazeutische Zusammensetzung nach Anspruch 1, wobei das Quellungsmittel ungefähr 3 Gew.-% bis ungefähr 6 Gew.-% der Zusammensetzung ausmacht.

17. Pharmazeutische Zusammensetzung nach Anspruch 1, wobei die Zusammensetzung des Weiteren ein Verdünnungsmittel enthält.

18. Pharmazeutische Zusammensetzung nach einem der vorhergehenden Ansprüche, wobei die Zusammensetzung in einer physikalischen Form ausgewählt aus der Gruppe bestehend aus Pellets, Kügelchen, Granalien, Tabletten, Trockensirup, Suspension und Kapseln geformt ist.

19. Pharmazeutische Zusammensetzung nach Anspruch 18, wobei die Festdosierungsform des Weiteren Binder, Gleitmittel, Anti-Haftmittel, Schmiermittel und Mischungen derselben enthält.

20. Pharmazeutische Zusammensetzung nach Anspruch 18, wobei die Tablettendosierungsform des Weiteren eine Beschichtung mit einem sich schnell auflösenden Film eines wasserlöslichen Polymers aufweist.

21. Pharmazeutische Zusammensetzung nach Anspruch 18, wobei die Dosierungsform als Trockensirup oder Suspension des Weiteren ein Pufferungsmittel, Konservierungsmittel, Anti-Verklumpungsmittel, Suspendierungsmittel, Anti-Schäummittel, Farbstoffe, Geschmackstoffe und Mischungen derselben aufweist.

22. Pharmazeutische Zusammensetzung nach Anspruch 18, wobei die Kapselhülle aus Gelatine, Hydroxypropylmethylcellulose oder Stärke hergestellt ist.

## Revendications

1. Composition pharmaceutique pour administration orale, qui comprend une quantité pharmaceutiquement efficace de cefpodoxime proxétyl absorbée sur un support pharmaceutique, dans laquelle le cefpodoxime proxétyl a une granulométrie moyenne inférieure à 30 micromètres et une répartition granulométrique de telle sorte qu'au moins 90% des particules sont inférieurs à 75 micromètres et la superficie est supérieure à 1,3 m²/g.

2. Composition pharmaceutique selon la revendication 1, dans laquelle le cefpodoxime proxétyl a une granulométrie moyenne inférieure à 20 micromètres et une répartition granulométrique de telle sorte qu'au moins 90% des particules sont inférieurs à 50 micromètres et la superficie est supérieure à 3,0 m²/g.

3. Composition pharmaceutique selon la revendication 1, dans laquelle le support pharmaceutique est un polymère hydrophile.

4. Composition pharmaceutique selon la revendication 3, dans laquelle le polymère hydrophile est un éther de cellulose, un polymère acrylique, une gomme naturelle et des mélanges de ceux-ci.

5. Composition pharmaceutique selon la revendication 4, dans laquelle l'éther de cellulose est choisi parmi le groupe composé de : hydroxypropylcellulose, hydroxypropylméthylcellulose, hydroxyéthylcellulose, méthylcellulose, hydroxyéthylméthylcellulose, hydroxypropyléthylcellulose, carboxyméthylcellulose, carboxyméthylcellulose de calcium, carboxyméthylcellulose de sodium, hydroxycellulose et mélanges de celles-ci.

6. Composition pharmaceutique selon la revendication 4, dans laquelle le polymère acrylique est choisi parmi le groupe composé de méthacrylates, copolymères de polyacrylate et de mélanges de ceux-ci.

7. Composition pharmaceutique selon la revendication 4, dans laquelle la gomme naturelle est choisie parmi le groupe composé de gomme de xanthane, gomme de karaya, gomme de caroube, gomme de guar, gomme de gélatine, gomme arabique, gomme adragante, carraghénine, pectine, agar, acide alginique, alginate de sodium et mélanges de ceux-ci.

8. Composition pharmaceutique selon la revendication 1, dans laquelle le polymère comprend environ 1 % à environ 60% en poids de ladite composition.

9. Composition pharmaceutique selon la revendication 1, dans laquelle le polymère comprend environ 1 % à environ 50% en poids de ladite composition.

10. Composition pharmaceutique selon la revendication 1, dans laquelle le support pharmaceutique comprend de plus un agent tensioactif.

11. Composition pharmaceutique selon la revendication 10, dans laquelle l'agent tensioactif est choisi parmi le groupe composé de laurylsulfate de sodium, copolymères de polyoxyéthylène-polyoxypropylène, polysorbats, esters de sorbitan et mélanges de ceux-ci.

12. Composition pharmaceutique selon la revendication 1, dans laquelle l'agent tensioactif comprend environ 1% à environ 5% en poids de ladite composition.

13. Composition pharmaceutique selon la revendication 1, dans laquelle la composition comprend également des agents de gonflement.

14. Composition pharmaceutique selon la revendication 13, dans laquelle l'agent de gonflement comprend un superdélitant.

15. Composition pharmaceutique selon la revendication 14, dans laquelle l'agent de gonflement est choisi parmi le groupe composé de polyvinylpyrrolidone réticulée, carboxyméthylcellulose sodique réticulée, glycolate d'amidon sodique et mélanges de ceux-ci.

16. Composition pharmaceutique selon la revendication 1, dans laquelle l'agent de gonflement comprend environ 3% à environ 6% en poids de ladite composition.

17. Composition pharmaceutique selon la revendication 1, dans laquelle la composition comprend de plus un diluant.

18. Composition pharmaceutique selon une quelconque des revendications précédentes, dans laquelle la composition est réalisée sous une forme physique choisie parmi le groupe composé de pastilles, pilules, granules, comprimés, sirop sec, suspension et gélules.

19. Composition pharmaceutique selon la revendication 18, dans laquelle la formulation solide comprend de plus un liant, un agent glissant, un agent antiadhérent, un agent lubrifiant, et des mélanges de ceux-ci.

20. Composition pharmaceutique selon la revendication 18, dans laquelle la formulation en comprimés comprend de plus un enrobage ayant une pellicule à dissolution rapide d'un polymère soluble dans l'eau.

21. Composition pharmaceutique selon la revendication 18, dans laquelle la formulation en sirop sec ou en suspension comprend un agent tampon, un conservateur, un agent antiagglomérant, un agent de suspension, un agent antimoussant, un aromatisant colorant et des mélanges de ceux-ci.

22. Composition pharmaceutique selon la revendication 18, dans laquelle l'enveloppe des gélules est réalisée en gélatine, hydroxypropylméthylcellulose ou amidon.
